(19) Europäisches Patentamt
European Patent Office
Office européen des brevets

(11) Numéro de publication: 0 214 014
A1

(12) # DEMANDE DE BREVET EUROPEEN

(21) Numéro de dépôt: 86401701.7

(22) Date de dépôt: 30.07.86

(51) Int. Cl.⁴: C 07 B 59/00
C 07 H 19/00

(30) Priorité: 01.08.85 FR 8511795

(43) Date de publication de la demande:
11.03.87 Bulletin 87/11

(84) Etats contractants désignés:
AT BE CH DE FR GB IT LI LU NL SE

(71) Demandeur: CENTRE NATIONAL DE LA RECHERCHE SCIENTIFIQUE (CNRS)
15, Quai Anatole France
F-75007 Paris (FR)

(72) Inventeur: Brahms, Georges
16, rue Vandrezanne
F-75644 Paris Cedex 13 (FR)

Dargouge, Olivier
Rua Sao Paolo, Lot 14 R/CA
P-2780 Oeiras (PT)

Quagliaroli, Daniel
8, rue Malet
F-93100 Montreuil (FR)

Vergne, Jacques
30, boulevard Diderot
F-91120 Palaiseau (FR)

Bardy, André
48, Résidence Les Gen ts
F-91420 Morangis (FR)

(74) Mandataire: Warcoin, Jacques et al
Cabinet Régimbeau 26, avenue Kléber
F-75116 Paris (FR)

(54) **Kit de marquage enzymatique de nucléosides triphosphates et procédé de préparation de nucléotides triphosphates marqués au 32P ou 35S au moyen de 32PO4H3 ou 35S-PO3Na3.**

(57) La présente invention concerne des kits de préparation de nucléotides triphosphates (NTP, d-NTP) ou de leurs dérivés au moyen de réactifs enzymatiques non spécifiques du nucléoside et des procédés simples mettant en oeuvre ces kits pour marquer avec les isotopes $^{32}P$ ou $^{35}S$ au moyen de $^{32}PO_4H_3$ ou $^{35}S$-$PO_3Na_3$ indifféremment en α,β, ou en γ des nucléotides.

EP 0 214 014 A1

**Description**

KIT DE MARQUAGE ENZYMATIQUE DE NUCLEOSIDES TRIPHOSPHATES ET PROCEDE DE PREPARATION DE NUCLEOTIDES TRIPHOSPHATES MARQUES AU $^{32}P$ OU $^{35}S$ AU MOYEN DE $^{32}PO_4H_3$ OU $^{35}S\text{-}PO_3Na_3$

La présente invention concerne un kit de synthèse enzymatique de nucléosides triphosphates (NTP) et un procédé de préparation de ces NTP marqués en α , β , ou en γ par des isotopes $^{32}P$ ou $^{35}S$.

Par NTP marqués en α , β ou γ on entend des nucléosides triphosphates, ou leurs dérivés dont un des trois phosphates ( α, β ou γ ) ( cf. Figure )a été substitué par un groupement porteur d'un isotope radioactif $^{32}PO_4$ ou $^{35}SPO_3$.

Les nucléosides triphosphates et leurs dérivés marqués sont utilisés pour de très nombreuses réactions de marquage, des acides nucléiques, ARN et ADN, ainsi que des protéines.

Les analogues phosphorothioates des nucléosides ont été utilisées dans un grand nombre (soixantaine) de réactions de phosphorylation des acides nucléiques et des protéines (cf. Eckstein (1983) Angew. Chem., 22, 423-439), ainsi le champ d'application des nucléotides marqués est extrêmement vaste ; on prévoit son extension en médecine et dans la pratique clinique.

La présente invention propose une préparation générale pouvant satisfaire toutes les demandes en ( α - $^{32}P$), ( β- $^{32}P$), et (γ - $^{32}P$)NTP ainsi qu'en ( α - $^{35}S$), ( β- $^{35}S$), et ( γ-$^{35}S$) NTP indépendamment de la nature du composé, c'est-à-dire indépendamment de la base ( les bases A, C, G, T, U, les bases modifiées, les bases rares ), qu'il s'agisse des dérivés méthylés, bromés, fluorés, par exemple, du sucre ou que le nucléoside soit un ribo- ou un déoxyribonucléoside.

L'absence de specificité permet d'utiliser le même nucléotide d'un bout à l'autre de la chaîne réactionnelle, ce qui distingue l'invention des approches enzymatiques connues, dont la spécificité limitée exigeait la présence d'ATP comme donneur de phosphate ; ainsi la préparation enzymatique selon l'invention permet d'éviter l'utilisation systématique d'ATP au cours de différentes étapes (cf. Walseth T. et R.A. Johnson (1979) B.BA 526 11-31, Schendel et Wells, (1973)J.B.C. 248, 8319-21 ; Reeve and Huang(1979) Nucl. Ac. Res. 6, 81).

La même préparation enzymatique permet l'incorporation soit d'un $^{32}P$ orthophosphate, soit d'un $^{35}S$ thiophosphate, $^{35}S\text{-}PO_3Na_3$, dont la préparation est décrite.

Le marquage au $^{35}S$ des NTP thiophosphates est particulièrement intéressant. En effet, l'isotope $^{35}S$ possède un temps de 1/2 vie relativement long (90 jours) et une énergie beta dix fois plus faible (0,167 MeV) par rapport à l'isotope $^{32}P$ d'énergie beta (1,7 MeV) et de temps de 1/2 vie de 15 jours.

Cette faible radioactivité des composés thiophosphoryles offre l'avantage d'une faible nocivité, ils sont donc d'un maniement plus aisé et place ce marquage dans une catégorie intermédiaire entre le matériel radioactif et non radioactif.

## ABREVIATIONS

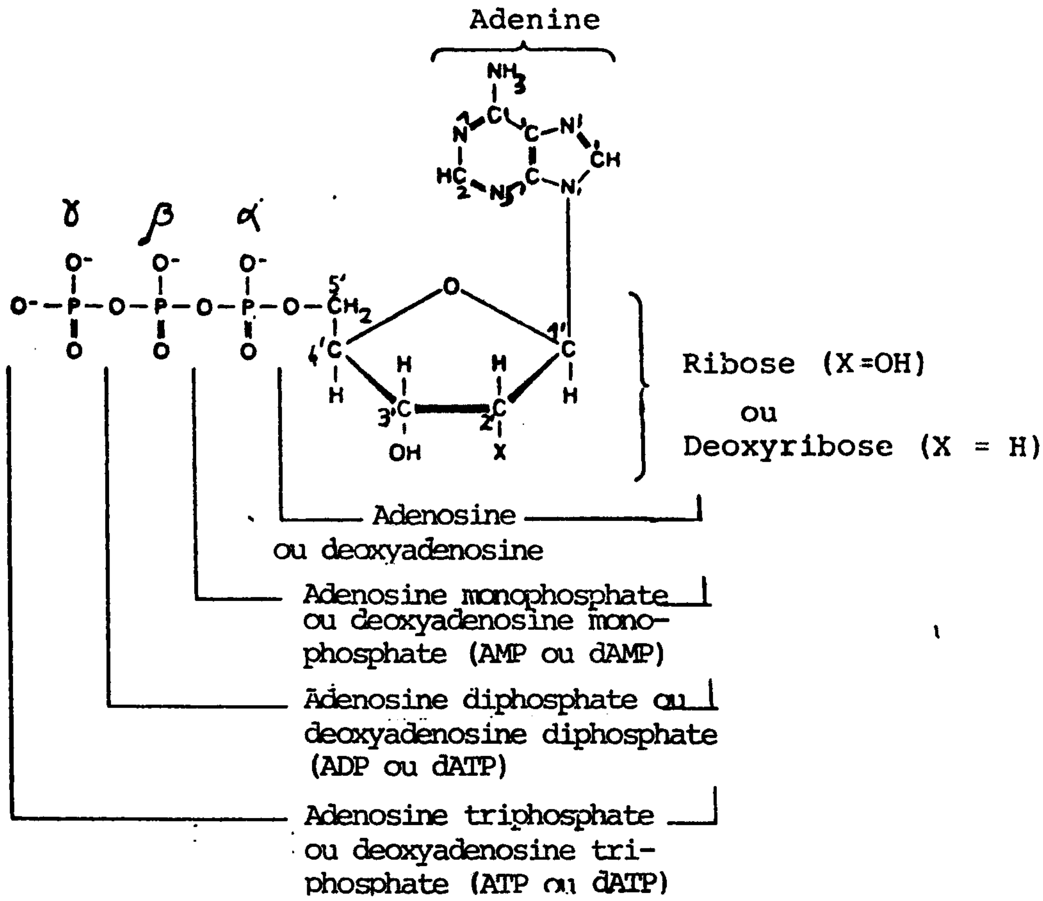


FORMULES DES NUCLEOSIDES MONO- DI- et TRIPHOSPHATE - NTP

De plus les ADN et les ARN thiosubstitués présentent une relativement plus importante résistance aux enzymes de dégradation par rapport aux phosphates correspondants.

La faible énergie dissipée par l'isotope $^{35}S$ rendant les phénomènes de radiolyse pratiquement inexistants, ainsi que la résistance aux nucléases des acides nucléiques thiosubstitués permet l'isolement d'ADN et d'ARN marqués, intact, in vivo comme in vitro (cf. Biggin et coll. (1983) PNAS USA 80, 3965 ; A. Radford (1983) Anal. Biochem. 134, 269-271).

Le marquage des ARN synthétisés au cours de la transcription peut s'effectuer en position 5' terminal au moyen de ( $\gamma$-$^{32}P$)NTP ou ($\gamma$ -$^{35}S$)NTP. Cependant, le problème majeur concerne la détection des ARN nouvellement synthétisés à cause des réactions de transfert du phosphoryl en $\gamma$ du NTP sur les ARN préexistants,catalysées par des kinases (Washington L.D. et Stallcup M.R. (1982) Nucl. Ac. Res. 10. 8311-8322). Par contre l'utilisation de ($\beta$ - $^{35}S$)NTP permet d'effectuer le marquage 5' terminal des ARN nouvellement initiées en excluant le transfert du phosphate radioactif. De plus, la présence d'un atome de soufre rend possible l'isolement des ARN néosynthétisés par chromatographie d'affinité sur colonne organo-mercurial (Hg agarose ou Hg sépharose par exemple) (Stallcup M.R. et Washington L.D. (1983) J. Biol. Chem. 283, 2803-2807).

La préparation des nucléotides ($\beta$ - $^{35}S$)NTP ouvre de larges perspectives dans l'étude de la synthèse des ARNs, en simplifiant une détection et leur purification.

Enfin, dans la séparation de matériel marqué au $^{35}S$ par les techniques électrophorétiques ou chromatographiques, la résolution des images d'autoradiographie est améliorée car le $^{35}S$ produit une diffusion radioactive moins importante que le$^{32}P$ lors de sa désintégration.

Par ailleurs, les résultats montrent que les préparations des NTP marqués obtenues selon l'invention sont les plus appropriées pour les études biologiques de la transcription et de la réplication, nick translation,

kination et pour les hybridations utilisant les protocoles de Southern, Norther, dot-blot ou cyto-blot (cf. Brahms J.G.. Dargouge O., Brahms S., Ohara Y., et Vagner V., (1985) J. Mol. Biol. 181, 455-465).

Le kit de préparation enzymatique de nucléotides triphosphates ou de leurs dérivés (NTP) marqués en α , β ou γ, selon l'invention est caractérisé en ce qu'il comporte un ou plusieurs des moyens suivants :

a) un moyen pour fixer en γ sur un NDP 5' un phosphoryledéterminé,

b) un moyen pour déplacer le phosphoryle déterminé du NTP 5', où ledit phosphoryle est en position γ , sur un NMP 3' où ledit phosphoryle se fixe en 5',

c) un moyen pour déphosphoryler en 3' le NDP 5' 3',

d) un moyen pour fixer deux phosphoryles 5' ( β et γ ) du NMP 5', et

e) un moyen pour déplacer le phosphoryle déterminé du NTP 5' (cf.a) où ledit phosphoryle est en γ sur un NMP 5' où ledit phosphoryle se fixe en β et phosphoryler le NDP 5' résultant en γ.

Par phosphoryle déterminé, on entend un phosphoryle porteur d'un isotope radioactif en particulier $^{32}P$ ou $^{35}S$ thiophosphate.

Ce kit permet en particulier la synthèse des NTP marqués au $^{32}P$ ou au $^{35}S$ en γ en une seule manipulation, dans un temps très court, de l'ordre de 90 minutes.

Le marquage en α s'effectue avec trois étapes supplémentaires, soit en une journée et demie.

Le marquage en β s'effectue en une étape supplémentaire, soit en 5 heures 10 minutes.

On obtient ainsi en 90 minutes ( γ ), une journée et demie ( α ) et 5 heures 10 minutes ( β ), par des manipulations simples, un produit brut marqué avec une excellente activité spécifique.

Le kit peut se présenter sous la forme d'un ensemble de réactifs enzymatiques indépendants nécessaire chacun à l'étape de marquage ou à une étape de la chaîne réactionnelle de marquage d'un NTP.

Chaque réactif est congelé rapidement, par exemple dans l'azote liquide et conservé à environ -70° C. Il est décongelé au moment de l'emploi.

La présente invention concerne également un procédé de préparation de nucléosides triphosphates ou de leurs dérivés marqués en α , β ou γ , qui comporte une étape de phosphorylation enzymatique en γ d'un NDP 5' par un agent de phosphorylation marqué, en donnant naissance au NTP 5' marqué en γ et, éventuellement, une ou plusieurs étapes ultérieures permettant le marquage en α ou en β . Sa mise en oeuvre peut être réalisée avec un kit selon l'invention.

Ainsi, la présente invention concerne un procédé de préparation enzymatique de nucléoside triphosphate ou de leurs dérivés (NTP) marqués en γ , caractérisé en ce que l'on traite à température ambiante un NDP 5' par un moyen pour fixer en γ sur un NDP 5' un phosphoryledéterminé en présence d'un agent de phosphorylation marqué, ce qui donne naissance au NTP 5'marqué en γ .

Pour marquer en γ un NTP, il suffit donc, pour réaliser l'étape

$$\text{NDP } 5' \xrightarrow{\text{RE1}} (\gamma\text{-}{}^{*}\text{P})\text{NTP } 5' \qquad (1)$$

où *P représente le marquage du résidu phosphoryl, de mettre en présence le réactif enzymatique capable de réaliser la phosphorylation en γ d'un NDP 5' (nucléotide diphosphate en 5') avec le NDP 5' portant la base du nucléotide triphosphate voulu et l'agent de phosphorylation marqué.

Comme réactif enzymatique selon l'invention, on utilise un réactif comportant, dans un tampon approprié, les enzymes :

Glycérol-Phosphate Deshydrogénase
Triose-Phosphate-isomérase
Glycéraldehyde-3-P-Deshydrogénase
3-P-glycérate kinase
Lactate Deshydrogénase

En particulier, on peut utiliser le réactif (RE 1) de composition :

Tris-Cl pH 9 10,5 mM
$MgCl_2$ 2 mM
DTT 12,5 mM
L-α-glycérolphosphate 0,25 mM
NAD+ 1 mM
Pyruvate (Na) 2mM
Glycérol-Phosphate-Deshydrogénase 70 µg/ml
Triose-P-isomérase 0,7 µg/ml
G-3-Phosphate-Deshydrogénase 70 µg/ml
3-Phosphate-glycérate kinase 416 µg/ml
Lactate Deshydrogénase 34,5 µg/ml

La réaction s'effectue à température ambiante et dure environ 1 heure 30 minutes.

Comme agent de phosphorylation, on peut utiliser tout orthophosphate marqué ou un thiophosphate marqué, par exemple $H_3\,{}^{32}PO_4$, $Na_3\,{}^{32}PO_4$, $Na_3\,PO_3\,{}^{35}S$.

La présente invention concerne également un procédé de préparation des NTP ou de leurs dérivés marqués en α , caractérisé en ce que:

a) dans une première étape, on traite à température ambiante un NDP 5' par un moyen pour fixer en γ

sur le NDP 5' un phosphoryl déterminé en présence d'un agent de phosphorylation marqué, dans des proportions adéquates, ce qui donne naissance au NTP 5' marqué en γ ,

b) on chauffe la solution résultante environ 2 minutes à environ 90° C,

c) dans une seconde étape on traite une quantité adéquate du NMP 3' de même base que le NDP 5' de départ par un moyen pour déplacer le phosphoryl déterminé du NTP 5', où ledit phosphoryl est en position γ , sur le NMP 3', où ledit phosphoryl se fixe en 5' en présence de la solution résultante de b), ce qui donne naissance au NDP 3'5' marqué en 5',

d) dans une troisième étape on traite la solution résultante de la seconde étape contenant le NDP 3'5' marqué en 5', par un moyen pour déphosphoryler en 3' un NDP 5' 3', dans des proportions adéquates, ce qui donne naissance au NMP 5' marqué en α ,

e) dans une quatrième étape, on traite la solution résultant de la troisième étape, contenant le NMP 5' marqué par un moyen pour fixer deux phosphoryls en β et γ d'un NMP 5', dans des proportions adéquates, ce qui donne naissance au NTP 5' marqué en α .

Pour réaliser le marquage en α, on réalise donc tout d'abord le marquage en γ (étape 1), puis trois étapes supplémentaires sont nécessaires, auxquelles correspondent trois réactifs enzymatiques.

Après l'étape de marquage en γ (étape 1), on réalise l'étape suivante :

( γ- *P)NTP + NMP 3' $\xrightarrow{RE2}$ (*P - 5')NDP 5', 3' (2)

Le réactif enzymatique RE2 déplace le résidu phosphoryl marqué du ( γ- *P)NTP sur la position 5' d'un NMP 3' ce qui donne naissance à un (*P - 5')NDP 3' 5' (nucléotide diphosphate en 3' et 5') marqué en 5', ceci en mélangeant à la solution résultant de l'étape 1 une quantité adéquate de RE2 et de NMP 3' (nucléotide monophosphate en 3') de même base que le NTP de départ.

Il est préférable de désactiver la solution resultant de l'étape 1 par un chauffage, par exemple pendant environ 2 minutes à environ 90° C.

Comme réactif RE2 selon l'invention, on utilise la polynucléotide kinase T4 ayant une activité de 5 à 10 unités.

Après avoir laissé agir le RE2, par exemple de 3 à 16 heures, on réalise l'étape

(*P-5')NDP 3'5' $\xrightarrow{RE3}$ *P NMP 5' (3)

Le réactif enzymatique RE3 déphosphoryle le NDP 3'5' en 3', ceci en ajoutant à la solution résultant de l'étape 2 une quantité adéquate de réactif RE3 pour les ribonucléotides. Comme réactif enzymatique pour l'étape 3, selon l'invention, on utilise l'enzyme nucléase $P_1$ à pH alcalin. Cette enzyme est en solution dans le tampon suivant :

- la nucléase $P_1$ se présente en solution dans Tris-Cl (20 µg de nucléase dans Tris-Cl 50 mM pour 25 µl de réactif pH 9,0) (RE3),

On réalise alors l'étape

*P NMP 5' $\xrightarrow{RE4}$ (α -*P)NTP (4)

Le réactif enzymatique permet la fixation de deux résidus phosphoryl sur le NMP 5' marqué, ceci en ajoutant une quantité adéquate de RE4 à la solution résultant de l'étape 3.

Comme réactif, pour l'étape 4, on utilise un réactif comportant, dans un tampon approprié, les enzymes :

Pyruvate kinase

NMP kinase (Nucléoside Monophosphate kinase)

(NDP kinase (Nucléoside Diphosphate Kinase))

On utilise le réactif (RE4) ayant la composition suivante :

Tris-Cl pH 8 50 mM

$MgCl_2$ 1 mM

DTT 5 mM

PEP 4 mM

KCl 4 mM

Pyruvate kinase 610 µg/ml

NMP kinase 2 500 µg/ml

(NDP kinase 510 µg/ml)

La synthèse est réalisée environ 4 heures après addition du réactif RE4, pour tous les nucléotides, après 1 heure pour l'ATP.

La présente invention concerne également un procédé de préparation des NTP ou de leurs dérivés marqués en β , caractérisé en ce que :

a) dans une première étape, on traite à température ambiante un NDP 5' par un moyen pour fixer en γ sur un NDP 5' un phosphoryl déterminé en présence d'un agent de phosphorylation marqué, dans des proportions adéquates, ce qui donne naissance au NTP 5' marqué en γ,

b) on chauffe la solution résultante 2 minutes à environ 90° C,

c) dans une seconde étape, on traite en quantités adéquates, du NMP 5' de même base que le NDP 5' de départ par un moyen pour déplacer un phosphoryl d'un NTP 5', où ledit phosphoryl est en γ sur un NMP 5' où ledit phosphoryl se fixe en β et phosphoryler le NDP 5' résultant, en présence de la solution résultant de b) contenant du NTP 5' marqué en γ, ce qui donne naissance au NTP 5' marqué en β .

Pour réaliser le marquage en β d'un NTP, on réalise donc d'abord le marquage en γ , puis une étape supplémentaire est nécessaire, que l'on réalise avec un réactif enzymatique RE4'.

( γ-*P)NTP + NMP-5' $\xrightarrow{RE4'}$ ( β -*P)NTP (4')

Comme réactif enzymatique,pour cette étape, on utilise un réactif comportant dans un tampon approprié, les enzymes :

Nucléoside monophosphate kinase

Pyruvate kinase.

On utilise le réactif (RE4') ayant la composition suivante :

Tris-Cl pH 8 50 mM

$MgCl_2$ 10 mM

KCl 4 mM

DTT 5 mM

PEP 4 mM

et.

Nucléoside Monophosphate kinase 1,25 mg/ml

Pyruvate kinase 300 µg/ml

Cette étape comporte deux stades :

$$(\gamma - {}^{*}P)NTP + NMP\ 5' \xrightarrow[NMPKinase]{} NDP\ 5' + (\beta - {}^{*}P)NDP\ 5'$$

pyruvate kinase ↓ (PEP → pyruvate)

$$(\gamma - {}^{*}P)NTP + NTP$$

et l'on a pu distinguer la réactivité des deux enzymes qui agissent successivement, comme cela apparaîtra dans les exemples.

Pour déceler la fin de la réaction enzymatique, à chaque étape, et définir le temps de réaction adéquat, on peut faire des chromatographies sur PEI-cellulose.

La synthèse des NTP marqués au $^{35}$ S se mène de la même façon que celle des NTP marqués au $^{32}P$, à l'agent de phosphorylation près. Ainsi, la synthèse des ( $\gamma$-$^{35}S$)NTP, ( $\beta$-$^{35}S$)NTP et ( $\alpha$-$^{35}S$)NTP consiste à incorporer un orthophosphate dont une molécule d'oxygène a été remplacée par une molécule de soufre (S).

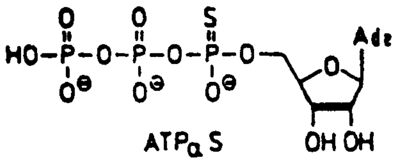

ATPαS

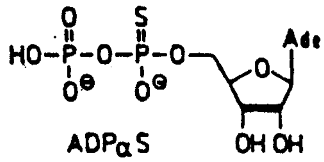

ADPαS

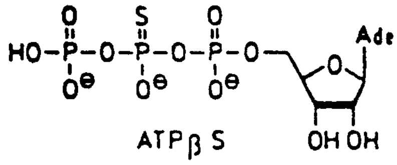

ATPβS

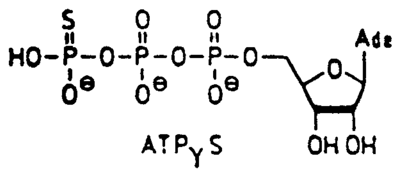

ATPγS

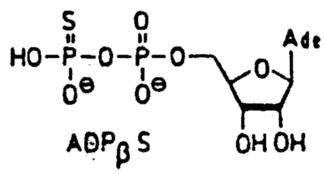

ADPβS

FORMULES DE L'ATP ET L'ADP PHOSPHOROTHIOATES S-ATP, S-ADP.

Les préparations sont identiques aux préparations effectuées pour le marquage au $^{32}P$. La différence est que l'agent phosphorylant marqué au $^{32}P$, par exemple $H_3\,^{32}PO_4$, normalement utilisé pour la première étape. est remplacé par une quantité équivalente de $Na_3PO^{35}S$ :

$Na_3PO_3\,^{35}S$ + NDP $\xrightarrow{RE1}$ (γ -$^{35}$S)NTP.

Le produit final de chaque marquage, marqué en γ , en β , ou en α, résultant de la dernière étape peut être purifié par chromatographie, liquide de haute performance sur une petite colonne $C^{18}$ jetable.

D'autres caractéristiques et avantages de l'invention apparaîtront dans les exemples ci-après et sur la figure annexée.

EXEMPLE 1 :

Synthèse de ( γ-$^{32}$P)UTP

On décongèle à température ambiante un aliquot du réactif enzymatique (RE1) (48 µl) et on ajoute 2 µl du nucléotide-5'-diphosphate UDP (2,5 mM). On mélange soigneusement :

Puis on ajoute : $^{32}P$ : 50 µl ($H_3$ $^{32}P$ $O_4$ dilué dans $H_2O$)

$H_2O$)

| | |
|---|---|
| RE1 | 48 µl |
| UDP | 2 µl |
| $^{32}P$ | 50 µl |
| TOTAL | 100 µl |

correspondant à 33 mCi (par exemple de 50 µCi à 5 mCi) en 6.6 mCi maximum pour 200 µl.

La réaction s'effectue à température ambiante pendant 1 heure 30 et est contrôlée par chromatographie sur PEI-cellulose ($KH_2PO_4$ 0,5 M pH 4).

Le produit final peut être soit purifié sur colonne jetable C 18, équilibrée avec $CH_3OH$ et TEAB 0.1 M. Le produit est élué avec 1 ml TEAB 0.1 M, -MeOH 80 %, après lavage avec 2 ml TEAB 0.1 M (durée de l'opération : 15 minutes). soit utilisé sans purification après avoir chauffé la solution résultant pendant 2 minutes à 90° C.

EXEMPLE 2 :

On a réalisé aussi, la synthèse de (γ -$^{32}$P)ATP et ( γ-$^{32}$P) CTP, GTP dans les mêmes conditions que pour l'exemple 1, à partir de ADP, CDP ou GDP.

On a contrôlé la réaction par chromatographie sur PEI-cellulose (polyéthylèneimine - cellulose).

La solution résultante peut être purifiée de la même façon qu'à l'exemple 1.

EXEMPLE 3 :

Synthèse de ( $\alpha$-$^{32}$P)UTP

On ajoute 20 µl du réactif enzymatique (RE2) préalablement décongelé (Polynucléotide kinase T4, 5-10 unités d'après les unités de Richardson (1965) PNAS USA 54, 158-163) et 15 mM de l'UMP 3', après avoir chauffé la solution résultant de l'exemple 1 sans purification pendant 2 minutes à 90° C.

On laisse agir 3 à 16 heures, puis on ajoute pendant 20 minutes 25 µl du réactif enzymatique (RE3) décongelé au dernier moment.

La synthèse se termine 4 heures après l'addition de 150 µl du réactif enzymatique (RE4) préalablement décongelé. On a testé chaque étape par chromatographie sur PEI-cellulose.

La solution résultante peut être purifiée comme décrit dans l'exemple 1.

EXEMPLE 4

Synthèse de ($\alpha$ -$^{32}$P)d-CTP

La synthèse se mène comme à l'exemple 3 jusqu'au réactif enzymatique (RE2). Ensuite on laisse agir 3 a 16 heures puis la synthèse se termine 4 heures après l'addition de 150 µl du réactif enzymatique (RE4) préalablement décongelé. On a contrôlé la réaction par chromatographie sur PEI-cellulose.

EXEMPLE 5

Synthèse de ( $\beta$ -$^{32}$P)ATP

On procède de la même façon que dans l'exemple 1, la réaction se déroule à 22° C pendant 1 heure 30.

Après avoir chauffé la solution obtenue à l'exemple 1 ( 2 minutes à 90° C), le volume est doublé par addition du réactif enzymatique (RE4') et de nucléotide 5'-monophosphate AMP 5' (2 µl d'une solution 2,5 mM). La réaction est complète au bout de 3 heures 30, à faible force ionique comme le montre la chromographie sur PEI-cellulose.

La deuxième étape a été séparée en deux parties pour montrer l'évolution des produits formés, et les produits décelés par chromatographie sur PEI-cellulose (tampon formiate pH 3.4) :

Avec la NMP kinase seulement, au bout de 30 minutes, on décèle la présence de ( $\beta$ -$^{32}$P)ADP et de ( $\gamma$ -$^{32}$P)ATP, au bout d'une heure, seule la présence de ($\beta$ - $^{32}$P)ADP est détectée.

Avec les deux enzymes, au bout d'une heure, on décèle ( $\beta$ -$^{32}$P)ATP seul.

EXEMPLE 6 :

On a procédé de la même façon qu'à l'exemple 5, pour ( $\beta$-$^{32}$P)UTP. La réaction a été vérifiée par chromatographie et dure 3 h 30.

EXEMPLE 7 :

Préparation du Monothiophosphate$^{35}$S radioactif ($Na_3PO_3{}^{35}S$)

Le but de la synthèse est la préparation en microquantité de monothiophosphate $S^{35}$ radiocatif sans entraîneur. Pour cela, on procède en deux étapes :

- dans un premier temps on prépare le chlorure de thiophosphoryl $P^{35}SCl_3$ ;
- dans un deuxième temps on utilise le $PS^{35}Cl_3$ synthétisé en présence de la soude NaOH pour effectuer la synthèse de $Na_3PO_3S^{35}$.

EXEMPLE 7a

Préparation du chlorure de Thiophosphoryl $S^{35}$ radioactif $P^{35}SCl_3$

Le chlorure de thiophosphoryl $PSCl_3$ est obtenu avec un excellent rendement par action directe de $^{35}S$ et $PCl_3$ en présence du catalyseur $AlCl_3$anhydre suivant la réaction :

$PCl_3 + AlCl_3 \longrightarrow PCl_3 \,.\, AlCl_3$

anhydre

$PCl_3 \,.\, AlCl_3$ composé d'association moléculaire, en présence du soufre $^{35}S$, se combine exothermiquement en régénérant le catalyseur

$PCl_3 \,.\, AlCl_3 + {}^{35}S \longrightarrow PS^{35}Cl_3 + AlCl_3$

La réaction a lieu dans les conditions anhydre dans un bain-marie d'huile où on peut chauffer d'abord à 40 -50 C pour obtenir la dissolution du soufre. Ensuite cette réaction exothermique atteint 120°C. La durée est de 5 à 10 minutes. $AlCl_3$ se dépose. On distille le $P^{35}SCl_3$ synthétisé. La technique employée permet que la réaction et la distillation aient lieu dans le même appareil.

Juste avant l'introduction des réactifs dans le vase réactionnel, on fait circuler un courant d'azote desséchant. L'ordre d'introduction des réactifs est le suivant :

1) $S^{35}$ soufre.

2) $AlCl_3$.

3) $PCl_3$.

Le $PCl_3$ étant très volatile et les quantités utilisées très faibles, est le réactif versé en dernier). On plonge ensuite le vase réactionnel dans le bain d'huile préchauffé à 50°-55°C pour obtenir la dissolution du soufre $^{35}S$. Lorsque la température s'élève, on observe une fumée blanche le soufre s'est dissous, on obtient une coloration jaune ; la distillation a lieu à 124°C. On recueille un distillat. Le rendement de la réaction est excellent grâce à un appareil adapté d'une part aux microquantités des produits radioactifs et d'autre part permettant un minimum de manipulations (par exemple changement de la colonne à reflux etc) ; on atteint ainsi 90 % à 95 %.

EXEMPLE 7 b

Préparation du Monothiophosphate $^{35}S$ radioactif, anhydre ($Na_3\ PO_3\ {}^{35}S$)

On a réalisé la synthèse de $Na_3PO_3{}^{35}S$ par la réaction :

$$P^{35}SCl_3 + 6\ NaOH \longrightarrow Na_3PO_3{}^{35}S + 3\ NaCl + 3\ H_2O$$

Mode opératoire

L'ordre d'introduction des réactifs dans le microdispositif réactionnel de chauffage est le suivant :

1) $H_2O$,
2) NaOH (à l'état solide),
3) $P^{35}S\ Cl_3$.

La dissolution des pastilles de soude dans l'eau provoque une élévation de température vers 50°C (réaction exothermique). Le $P^{35}SCl_3$ introduit se dépose au fond du microrecipient de chauffage équipé d'un reflux. On chauffe à température constante aux alentours de 105°C pour 15 minutes ou plus.

En fin de réaction, la couche de $P^{35}SCl_3$ disparaît et le produit formé est précipité en refroidissant le mélange dans un bain d'eau et de glace. Le produit en microquantité $Na_3PO_3{}^{35}S$ contenu dans le récipient réactionnel est cristallisé en utilisant un procédé original de centrifugation. On centrifuge le précipité pendant 10 minutes à 10 000 tours/min et on élimine le surnageant avec les impuretés. On peut redissoudre le précipité dans $H_2O$ dans un bain marie à ~ 50°C et on répète l'opération en centrifugeant à nouveau pour éliminer les impuretés dans le culot et on récupère le surnageant. On recristallise directement dans le même récipient en ajoutant du méthanol.

Le produit est cristallisé et on centrifuge 10 minutes à 10 000 tours/min, on élimine le surnageant, on ajoute ensuite du méthanol en agitant pour laver les cristaux. On effectue cette opération trois fois. Après la dernière recristallisation, le surnageant est éliminé et le produit cristallisé dans le récipient (dans le même tube), est désséché sous une cloche à vide (pour l'évaporation du solvant).

Par ce procédé, des microquantités du $Na_3PO_3{}^{35}S$ radioactif anhydre peuvent être recueillies après les trois cristallisations avec un rendement dépassant 75 %.

EXEMPLE 8 :

Synthèse de ( $\gamma$-$^{35}S$), ($\beta$ -$^{35}S$), ( $\alpha$-$^{35}S$)NTP

On remplace, dans la première étape qui fait intervenir le réactif enzymatique 1, l'acide ortophosphorique $H_3\ PO_4$ marqué au $^{32}P$ par une quantité équivalente de $Na_3\ PO_3\ S$ marqué au $^{35}S$, et on opère de la même façon qu'aux exemples 1 à 6.

Afin de suivre l'évolution de la réaction, les essais ont été réalisés en présence d'ADP-$^{14}C$, et de thiophosphate synthétisé au laboratoire.

1) la figure 1 présente la cinétique comparée de la synthèse d'ATP à partir d'ADP-$^{14}$ C en présence de $Na_3PO_3S$ et de $Na_3PO_4$ dans différentes conditions. Sur cette figure, on a représenté en fonction du temps

$$\text{(en heures)} \quad \frac{ATP}{ATP + ADP} \times 100 \text{ pour ;}$$

$Na_3PO_4$ dans $H_2O$ ○
$Na_3PO_4$ dans HCl pH 4,0 □
$Na_3PO_3S$ dans $H_2O$ ●
$Na_3PO_3S$ dans HCl pH 4,0 ■

La vitesse initiale de la réaction est diminuée en présence de $Na_3PO_3S$ mais le maximum d'incorporation est la même dans les deux cas.

2) On a procédé à l'identification des produits synthétisés en fonction du temps par chromatographie sur HPLC et sur PEI cellulose dans différentes conditions. Les différents tampons utilisés pour diluer $Na_3PO_3S$ ne semblent pas influencer la synthèse et la proportion d'ATP(S) synthétisé au bout de 2 heures

indique une bonne stabilité du $Na_3PO_3S$ dans le milieu réactionnel.

## Revendications

1. Kit de préparation enzymatique de nucléosides triphosphates NTP et dNTP ou de leurs dérivés, non spécifique du nucléoside (base et sucre), pour le marquage en α, β , ou γ , caractérisé en ce qu'il comporte un ou plusieurs des moyens suivants :

a) un moyen pour fixer en γ sur un NDP 5' un phosphate déterminé,

b) un moyen pour déplacer le phosphoryl déterminé du NTP 5', où ledit phosphoryl est en position γ , sur un NMP 3' où ledit phosphoryl se fixe en 5',

c) un moyen pour déphosphoryler en 3' le NDP 5' 3',

d) un moyen pour fixer deux phosphoryls en 5' du NMP 5', et

e) un moyen pour déplacer le phosphoryl déterminé du NTP 5', où ledit phosphoryl est en γ sur un NMP 5' où ledit phosphoryl se fixe en β et phosphoryler le NDP 5' résultant.

2. Kit de préparation enzymatique selon la revendication 1, caractérisé en ce qu'il comporte, pour le marquage en γ , le moyen défini en a).

3. Kit de préparation enzymatique selon la renvendication 2, caractérisé en ce qu'il comporte en outre, pour le marquage en α , les moyens définis en b), c) et d).

4. Kit de préparation enzymatique selon la revendication 2, caractérisé en ce qu'il comporte en outre pour le marquage en β , le moyen défini en e).

5. Kit de préparation enzymatique selon l'une des revendications 1 à 4, caractérisé en ce que le moyen défini en a) comporte un réactif enzymatique incluant :

Glycérol-3-Phosphate Deshydrogénase
Triosephosphate isomérase
Glycéraldéhyle-3-Phosphate Deshydrogénase
3-Phosphoglycérate kinase
Lactate Deshydrogénase

6. Kit de préparation enzymatique selon l'une des revendications 1 à 5, caractérisé en ce que le moyen défini en a) est un réactif enzymatique ayant la composition suivante :

Glycérol-3-Phosphate Deshydrogénase 70 µg/ml
Triosephosphate isomérase 0,7 µg/ml
Glycéraldehyle-3-Phosphate Deshydrogénase 70 µg/ml
3-Phosphoglycérate kinase 416 µg/ml
Lactate deshydrogénase 34,5 µg/ml
Tris HCl pH 9 10,5 mM
$MgCl_2$ 2 mM
DTT 12,5 mM
L-Glycérolphosphate 0,25 mM
NAD 1 mM
Pyruvate (Na) 2 mM

7. Kit de préparation enzymatique selon l'une des revendications 1 à 6, caractérisé en ce que le moyen défini en b) est un réactif enzymatique incluant :

Polynucléotide kinase T4
3'-Nucléotide monophosphate.

8. Kit de préparation enzymatique selon la revendication 7, caractérisé en ce que le moyen défini en b) est un réactif enzymatique ayant la composition suivante :

Polynucléotide kinase T4 0,25-0,5 u/µl
3' -Nucléotide monophosphate 15 mM

9. Kit de préparation enzymatique selon l'une des des revendications 1 à 8, caractérisé en ce que le moyen défini en c) est un réactif enzymatique comportant la nucléase $P_1$ dans un tampon approprié.

10. Kit de préparation enzymatique selon l'une des revendications 1 à 9, caractérisé en ce que le moyen défini en d) est un réactif enzymatique comportant, dans un tampon approprié les enzymes :

Pyruvate kinase
NMP kinase
NDP kinase

11. Kit de préparation enzymatique selon l'une des revendications 1 à 9, caractérisé en ce que le moyen défini en d) est un réactif enzymatique ayant la composition suivante :

Tris-Cl pH 8 50 mM
$MgCl_2$ 1 mM
DTT 5 mM
PEP 4 mM
KCl 4 mM
Pyruvate kinase 600 µg/ml

NMP kinase 2.5 mg/ml
NDP kinase 500 µg/ml

12. Kit de préparation enzymatique selon l'une des revendications 1 à 11, caractérisé en ce que le moyen défini en e) est un réactif enzymatique comportant dans un tampon approprié, les enzymes :
Nucléotidemonophosphate-kinase
Pyruvate kinase

13. Kit de préparation enzymatique selon l'une des revendications 1 à 12, caractérisé en ce que le moyen défini en e) est un réactif enzymatique ayant la composition suivante :
Pyruvate kinase 600 µg/ml
NMP kinase 2500 µg/ml
(NDP kinase 510 µg/ml
Tris HCl pH 8-7,8 50 mM
$MgCl_2$ 10 mM
DTT 5 mM
KCl 4 mM
PEP 4 mM

14. Procédé de préparation enzymatique de nucléoside triphosphate (NTP, d-NTP) ou de leurs dérivés marqués en γ . caractérisé en ce que l'on traite à température ambiante un NDP 5' par le moyen défini à la revendication 1a) en présence d'un agent de phosphorylation marqué, ce qui donne naissance au NTP 5' marqué en γ.

15. Procédé de préparation enzymatique de nucléotides triphosphates ou de leurs dérivés (NTP, d-NTP) marqués en α, caractérisé en ce que :

a) dans une première étape, on traite à température ambiante un NDP 5' par le moyen défini à la revendication 1a) en présence d'un agent de phosphorylation marqué, ce qui donne naissance au NTP 5' marqué en γ ,

b) on chauffe la solution résultante 2 minutes à 90° C,

c) dans une seconde étape on traite une quantité adéquate du NMP 3' de même base que le NDP 5' de départ par le moyen défini à la revendication 1b) en présence de la solution résultante de b), ce qui donne naissance au NDP 3'5' marqué en 5',

d) dans une troisième étape on traite la solution résultante de la seconde étape contenant le NDP 3'5' marqué en 5', par le moyen défini à la revendication 1c), dans des proportions adéquates. ce qui donne naissance au NMP 5' marqué en α ,

e) dans une quatrième étape, on traite la solution résultante de la troisième étape, contenant le NMP 5' marqué par le moyen défini à la revendication 1d), dans des proportions adéquates. ce qui donne naissance au NTP 5' marqué en α .

16. Procédé de préparation enzymatique de nucléotides triphosphates (NTP-dNTP) ou de leurs dérivés marqués en β , caractérisé en ce que :

a) dans une première étape, on traite à température ambiante un NDP 5' par le moyen défini à la revendication 1a) en présence d'un agent de phosphorylation marqué, ce qui donne naissance au NTP 5' marqué en γ,

b) on chauffe la solution résultante 2 minutes à 90°C,

c) dans une seconde étape, on traite en quantités adéquates, du NMP 5', de même base que le NDP 5' de départ par le moyen défini à la revendication 1e), en présence de la solution résultante de b) contenant du NTP 5' marqué en γ , ce qui donne naissance au NTP 5' marqué en β.

17. Procédé selon l'une des revendications 14 à 16, caractérisé en ce que l'agent de phosphorylation marqué est $(^{32}PO_4)^{---}$ .

18. Procédé selon l'une des revendications 14 à 16, caractérisé en ce que l'agent de phosphorylation marqué est $(PO_3\ ^{35}S)^{---}$ .

19. Procédé selon l'une des revendications 14 à 18, caractérisé en ce qu'en outre l'on purifie la solution obtenue par chromatographie.

20. Procédé selon l'une des revendications 14 à 19, caractérisé en ce que le produit obtenu peut être purifié sur une petite colonne de chromatographie liquide de haute performance jetable.

– Leerseite –

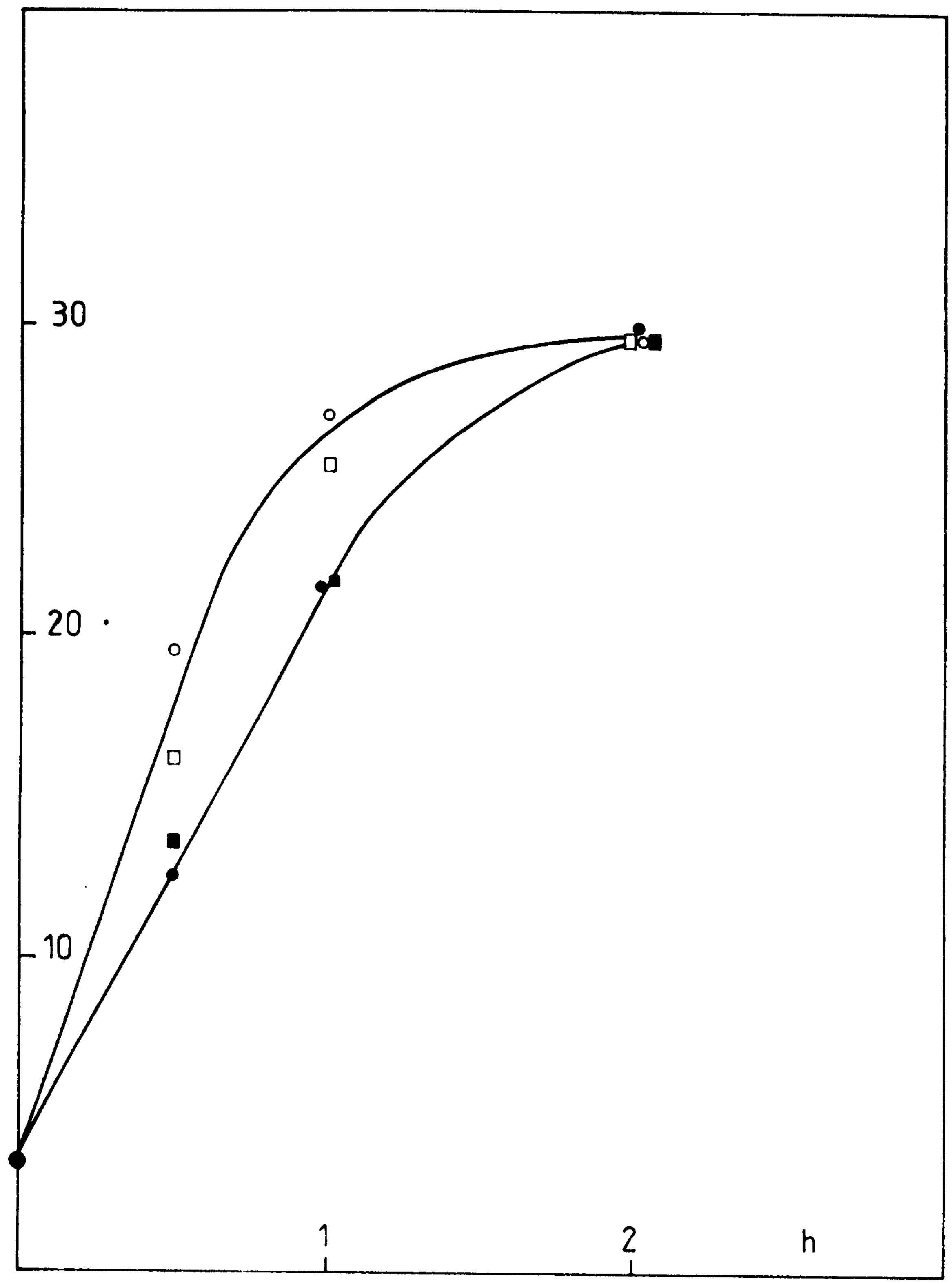


FIG. 1

Office européen des brevets

# RAPPORT DE RECHERCHE EUROPEENNE

Numero de la demande

EP 86 40 1701

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| Y | US-A-4 209 589 (R.A. JOHNSON et al.)<br>* En entier *<br>--- | 1,5,6 | C 07 B 59/00<br>C 07 H 19/00 |
| Y | DD-A- 212 391 (E. BAUSCHKE et al.)<br>* En entier *<br>--- | 1,5,6 | |
| A | CHEMICAL ABSTRACTS, vol. 90, 1979, page 295, résumé no. 182689v, Columbus, Ohio, US; A.E. REEVE et al.: "A method for the enzymic synthesis and purification of [alpha-32P]-nucleoside triphosphates", & NUCLEIC ACIDS RES. 1979, 6(1), 81-90<br>--- | 1,8 | |
| | | | DOMAINES TECHNIQUES RECHERCHES (Int Cl 4) |
| A | CHEMICAL ABSTRACTS, vol. 90, 1979, page 297, résumé no. 182709b, Columbus, Ohio, US; T.F. WALSETH et al.: "The enzymic preparation of [alpha-32P]-nucleoside triphosphates, cyclic [32P]-AMP, and cyclic [32P]-GMP", & BIOCHIM. BIOPHYS. ACTA 1979, 562(1), 11-31<br>--- -/- | 1,7 | C 07 B 59/00<br>C 07 H 19/00 |

Le present rapport de recherche a été etabli pour toutes les revendications

| Lieu de la recherche | Date d'achevement de la recherche | Examinateur |
|---|---|---|
| LA HAYE | 12-11-1986 | VAN BIJLEN H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié a la date de dépôt ou après cette date
D : cité dans la demande
L : cité pour d'autres raisons

& : membre de la même famille, document correspondant

OEB Form 1503 03 82

Office européen des brevets

# RAPPORT DE RECHERCHE EUROPEENNE

Numero de la demande

EP 86 40 1701



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | CHEMICAL ABSTRACTS, vol. 100, 1984, page 327, résumé no. 171204g, Columbus, Ohio, US; & IL-A-60 707 (YEDA RESEARCH AND DEVELOPMENT CO. LTD.) 30-09-1983 | 1,7 | |
| | ----- | | |

DOMAINES TECHNIQUES RECHERCHES (Int Cl 4)

Le present rapport de recherche a eté établi pour toutes les revendications

| Lieu de la recherche | Date d achèvement de la recherche | Examinateur |
|---|---|---|
| LA HAYE | 12-11-1986 | VAN BIJLEN H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82